# EUROPEAN PATENT SPECIFICATION

(11) **EP 3 077 811 B1**
(45) Date of publication and mention of the grant of the patent: **05.09.2018**
(21) Application number: 14824767.9
(22) Date of filing: 02.12.2014
(51) Int. Cl.: G01N 33/36, G01N 17/00, G01N 21/64

(54) **A METHOD AND DEVICE FOR FATIGUE TESTING OF PHOTOCHROMIC, FLUORESCENT OR PHOSPHORESCENT DYES**
VERFAHREN UND VORRICHTUNG ZUR ERMÜDUNGSPRÜFUNG VON PHOTOCHROMEN, FLUORESZIERENDEN ODER PHOSPHORESZIERENDEN FARBSTOFFEN
PROCÉDÉ ET DISPOSITIF D'ESSAI DE FATIGUE DE COLORANTS PHOTOCHROMES, FLUORESCENTS OU PHOSPHORESCENTS

(30) Priority: 03.12.2013 CZ 20130959
(43) Date of publication of application: 12.10.2016
(73) Proprietor: Technicka Univerzita v Liberci, 461 17 Liberec (CZ)
(72) Inventor: VIK, Michal Doc. Ing., PhD, 460 01 Liberec (CZ); VIKOVA, Martina, Ing., PhD, 460 01 Liberec (CZ)
(74) Representative: Musil, Dobroslav
(86) International application number: PCT/CZ2014/000144
(87) International publication number: WO 2015/081918

(56) References cited:
- MARTINA VIKOVA: "PHOTOCHROMIC TEXTILES", PHD THESIS TO HERIOT-WATT UNIVERSITY, 21 March 2012 (2012-03-21), pages 1-199, XP002736786, Retrieved from the Internet: URL:http://hdl.handle.net/10399/2439 [retrieved on 2015-03-04]

## Description

### Technical field

The invention relates to a method for fatigue testing of photochromic, fluorescent or phosphorescent dye/dyes or of a mixture of at least two of them.

The invention further relates to a device for carrying out this method.

### Background art

For monitoring changes in different characteristics of different materials as a result of long-term fatigue stress, the so-called accelerated ageing tests are currently used, in which long-term impact of external conditions/factors is simulated in a relatively short time by exposing the particular materials to these conditions/factors cyclically with intensified influence (such as higher intensities of radiation, higher temperatures, increased concentrations of ozone, etc.). After carrying out a predetermined number of cycles, changes in the characteristics of the materials or changes in their responses to a particular external condition/factor are monitored and evaluated. These tests include, for example, also tests of color fastness of textile dyes under exposure to light in accordance with the CSN (Czech Technical Standard) EN ISO 105-B04 "Textiles - Tests for color fastness - Part B04: Color fastness to artificial weathering: Xenon arc fading lamp test" and CSN (Czech Technical Standard) EN ISO 105-B06 "Textiles - Tests for color fastness - Part B06: Color fastness and ageing at artificial light at high temperatures: Xenon arc fading lamp test". However, their disadvantage consists in that they cannot be used for fatigue testing of photochromic, fluorescent or phosphorescent dye/dyes or their mixture, since in the case of these dyes, these tests lead to an incorrect conclusion about their low color fastness. This is caused primarily by the fact that during the exposure of most photochromic dyes to intense artificial light, a triplet-triplet excitation occurs relatively quickly, followed by degradation of their chromic system, resulting in their bleaching.

For these reasons, several new methods and devices have been developed for fatigue testing of photochromic, fluorescent or phosphorescent dye/dyes or of their mixture, which are based on using a light source having an output and intensity which closely match the characteristics of natural daylight. Nevertheless, their disadvantage lies in the fact that they take place in a discontinuous way and are therefore rather time-consuming. Also, the technical equipment used, especially the light source, as well as the manner of its operation, when the monitored sample is lit by short flashes of light, do not allow illumination to pass through the entire sample properly, and so initially the response of the monitored dye/dyes occurs only in a thin layer on the surface of the sample, which is another drawback. Due to this, several initial responses or rather several tens of initial responses of the dye/dyes are significantly distorted.

A known system for fatigue testing of photochromic prints is disclosed in "PHOTOCHROMIC TEXTILES", PHD THESIS TO HERIOT-WATT UNIVERSITY, 21 March 2012 (2012-03-21), pages 1-199 attributed to Martina Vikova. The goal of the invention is to devise a method for fatigue testing of photochromic, fluorescent or phosphorescent dye/dyes or of a mixture of at least two of them, which would eliminate the disadvantages of the background art and enable reliable testing of this/these dye/dyes from the very beginning of the test.

The aim of the invention is also to provide a device for carrying out this method.

### Principle of the invention

The goal of the invention is achieved by a method for fatigue testing of photochromic, fluorescent or phosphorescent dye/dyes or of a mixture of at least two of them, whose principle consists in that a sample containing photochromic, fluorescent or phosphorescent dye/dyes or a mixture of at least two of them is exposed to a predetermined number of cycles of luminous exposure to an excitation light beam, which evokes a color response of the photochromic, fluorescent or phosphorescent dye/dyes or of the mixture of at least two of them in the sample, whereby before and/or during and/or after each predetermined exposure to the excitation light beam, the sample containing photochromic, fluorescent or phosphorescent dye/dyes, or of the mixture of at least two of them is exposed at least once to a polychromatic exposure light beam, due to which the dye/dyes is/are subject to fatigue loading. Simultaneously, a measuring light beam is introduced to the sample and is then reflected from it, whereby the change and/or the course of the change in the characteristics of the measuring light beam reflected from the sample is monitored by a spectrometer and from this change and/or the course of the change it is possible to deduce the course of the color response and/or the change in the color response of the particular photochromic, fluorescent or phosphorescent dye/dyes or of the mixture of at least two of them to the exposure to the excitation light beam, and, consequently, it is possible to deduce the fatigue of this/these photochromic, fluorescent or phosphorescent dye/dyes, or of the mixture of at least two of them.

The exposure light beam simulates advantageously natural daylight.

In addition to that, the goal of the invention is also achieved by a device for fatigue testing of photochromic, fluorescent or phosphorescent dye/dyes, or of a mixture of at least two of them, whose principle consists in that it contains a spherical optical integrator, in which a measuring aperture, an inlet aperture of the measuring light beam, an inlet aperture of the exposure and excitation light beams and an outlet aperture of the measuring light beam are formed. Moreover, in the vicinity of the measuring aperture, outside the optical integrator, is arranged space for storing and fixing the sample containing the tested photochromic, fluorescent or phosphorescent dye/dyes, or the mixture of at least two of them. Outside the optical integrator, the inlet aperture of the measuring light beam is aligned with a source of the measuring light beam, whereby a filter of luminous radiation having an excitation wavelength, as well as a filter of infrared radiation, are positioned in the path of the measuring light beam. To the inlet aperture of the exposure and excitation light beams is outside the optical integrator assigned a path of the exposure light beam containing a polychromatic light source, and a path of the excitation light beam containing a source of the excitation light beam. Moreover, both these paths have a common portion containing a beam splitter arranged in an inverse arrangement, a chopper and mirror optics for introducing the exposure and excitation light beams into the inner space of the optical integrator, whereby the path of the exposure light beam contains a shutter of the exposure light beam, whereas the path of the excitation light beam contains a shutter of the excitation light beam and a monochromator. To the outlet aperture of the measuring light beam is outside the optical integrator assigned a path of the measuring light beam containing a spectrometer. The path contains a chopper and mirror optics for introducing the measuring light beam into the sensing space of the spectrometer.

It is also advantageous if the path of the exposure beam includes at least one correction filter which serves to filter off certain irrelevant or ballast components of the exposure light beam and to approximate the character of this beam to the character of light according to the required conditions of testing, for example, to natural daylight at particular times of the day, etc.

So as to obtain more accurate results, it is advantageous if the path of the measuring light beam includes an objective which serves to focus on the active spot of the sample where the response of its dye/dyes takes place and/or to adjust the size of the area of the sample that is being sensed by the spectrometer.

In order to achieve the required excitation of the color change in the photochromic, fluorescent or phosphorescent dye/dyes or the mixture of at least two of them, as well as the fatigue loading of the sample by the exposure light beam, in a manner that is as accurate as possible, it is advantageous if the chopper in the common part of the path of the exposure and excitation light beams and the chopper in the path of the measuring light beam are interconnected by a time bond. For the same reason, it is also favourable if the shutter of the exposure light beam is provided with a time lock and is interconnected with the shutter of the excitation light beam by a time bond.

So as to be able to monitor the influence of the temperature on the fatigue testing of the photochromic and/or fluorescent and/or phosphorescent dye/dyes, or, on the contrary, to exclude completely the influence of the temperature or its change from the fatigue testing, all the parts of the device for the fatigue testing of the photochromic, fluorescent or phosphorescent dye/dyes, or at least the space for storing and fixing the sample, are mounted in a thermostatic box. Preferably, a thermostatic head is also mounted in the vicinity of the space for storing and fixing the sample, controlling the temperature of this space and of the sample stored in it and, should the need arise, amending the temperature as well.

Furthermore, any part of the path of the exposure light beam and/or that of the excitation light beam and/or that of the measuring light beam can consist of an optical fiber/optical fibers.

In order to avoid the so-called drift of the luminous sources, the device for fatigue testing of photochromic, fluorescent or phosphorescent dye/dyes, or of a mixture of at least two of them, according to the invention, is preferably designed as a two-beam device, which means that it is provided with an unillustrated source of a reference light beam, with which is aligned a reference spectrometer which measures the reflection of the reference light beam from the constant reference sample having a known value of absolute reflectance, which is a white standard or, for example, a part of the white inner surface of the optical integrator.

### Description of drawings

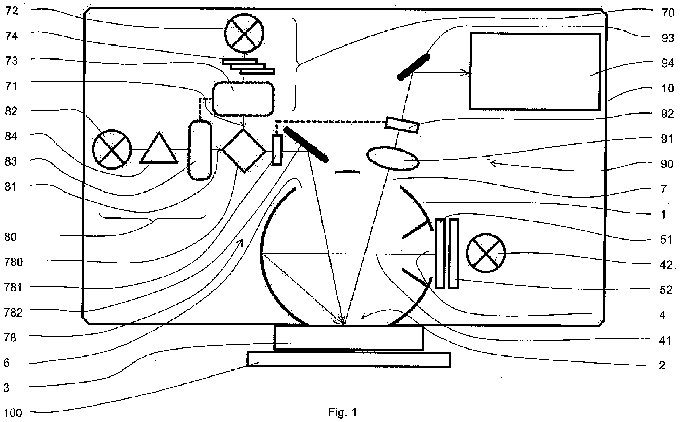

In the enclosed drawing, Fig. 1 shows a diagram of one variant of a device for carrying out fatigue testing of photochromic, fluorescent or phosphorescent dye/dyes, or of a mixture of at least two of them according to the invention.

### Examples of embodiment

A method for fatigue testing of photochromic, fluorescent or phosphorescent dye/dyes or of a mixture of at least two of them according to the invention will be explained using a concrete variant of a device for carrying out this method, shown in Fig. 1. However, this variant is just one among a number of possible alternatives of embodiment of this device, whereby other alternatives of embodiment may differ from this variant, for example the spatial arrangement of individual elements may vary, as well as the method for transmission/link of the excitation and/or exposure and/or measuring light beam, whereby any of the beams may be at least on part of its path transmitted/carried, for example, through an optical fiber/optical fibers, or using any other known method, etc.

A device for fatigue testing of photochromic, fluorescent or phosphorescent dye/dyes or of a mixture of at least two of them according to the invention, shown in Fig. **1****,** contains an optical integrator **1** of a spherical shape. This optical integrator **1**, due to the material from which it is made and/or due to its surface finish, such as a coat of paint, is impermeable to light radiation, and therefore it prevents ambient light penetration into the inner space, thus excluding its influence on the course of the fatigue testing of the particular photochromic, fluorescent or phosphorescent dye/dyes or the mixture of at least two of them, prevents the excitation, exposure and measuring light beams, which are introduced to the inner space in a controlled manner, from dispersing into the ambient surroundings, and at the same time enables to measure the intensity of the luminous radiation reflected independently of the direction taken by the radiation after being reflected.

A measuring aperture **2** is formed in the lower portion of the optical integrator **1.** In its close vicinity, outside the optical integrator **1,** is arranged unillustrated space for storing and fixing the sample **3** containing the tested photochromic, fluorescent or phosphorescent dye/dyes or the mixture of at least two of them. The sample **3** is, for example, a textile, but substantially any material could be used, and the dye being tested is any photochromic, fluorescent or phosphorescent dye/dyes or a mixture of at least two of them, which is sensitive, for example, to UV radiation.

Above the measuring aperture **2,** in the central portion of the optical integrator **1,** is formed an inlet aperture **4** of a measuring light beam **41,** with which is outside the optical integrator **1** aligned a source **42** of this measuring light beam **41.** In the path of the measuring light beam **41** is arranged a filter **51** of luminous radiation having an excitation wavelength, which is in this particular case UV radiation, and a filter **52** of IR radiation, due to which the measuring light beam **41** does not cause the excitation of the photochromic, fluorescent or phosphorescent dye/dyes or of the mixture of at least two of them in the sample **3,** nor does it contribute to it in any manner. It does not heat the inner space of the optical integrator **1** and/or the sample **3** either.

An inlet aperture **6** of the excitation and of the exposure light beams, as well as an outlet aperture **7** of the measuring light beam **41** are formed in the upper portion of the optical integrator **1.** To the inlet aperture **6** of the excitation and of the exposure light beams **71, 81** is outside the optical integrator **1** assigned a path **70** of the exposure light beam **71** with a source **72** of the exposure light beam **71,** and the path **80** of the excitation light beam **81** with a source **82** of the excitation light beam **81.** Furthermore, both these paths **70** and **80** have a common part **78** which contains a beam splitter **780,** a chopper **781** and mirror optics **782** and which introduces the exposure and excitation light beams **71, 81** through the inlet aperture **6** into the inner space of the optical integrator **1.** The path **70** of the exposure light beam **71** further contains a shutter **73** of the exposure light beam **71** and a correction filter **74/**correction filters, assigned to the source **72** of the exposure light beam **71,** while the path **80** of the excitation light beam **81** contains a shutter **83** of the excitation light beam **81** and a monochromator **84,** assigned to the source **82** of the excitation light beam **81.**

The mirror optics **782,** comprising a mirror or a system of mirrors, serves to direct the exposure and excitation light beams **71, 81** into the inner space of the optical integrator **1,** without decreasing their intensity or changing their characteristics. Moreover, it enables to screen off their IR component, which is vital for achieving the required accuracy of the fatigue testing, since the IR component of any of them could otherwise cause heating the inner space of the optical integrator **1** and/or the sample **3,** which could lead at least in the case of some photochromic, fluorescent or phosphorescent dyes to undesired additional excitation or could influence the parameters that are measured.

A chopper **781,** which is a synchronous system of periodical blocking the optical path of the beam light, is used to set a cyclic passage of the exposure and/or excitation light beams **71, 81**, and thus to control the excitation and reversal phases of the photochromic, fluorescent or phosphorescent dye/dyes, or of the mixture of at least two of them in the sample **3,** and its loading by the exposure light beam **71.** The chopper **781** can be electronic or mechanical, whereby an advantageous embodiment contains a mechanical chopper **781,** which is designed as a divided circular screen which by regulating its revolutions enables to change the period of time during which the light beam, in this particular case the exposure and/or excitation light beams **71, 81,** is let through or, on the contrary, blocked.

A beam splitter **780** of any known type, preferably a mirror-type beam splitter, is in the common part **78** of the path of the exposure and excitation fight beams **71, 81** arranged inversely, and so it fuses the exposure light beam **71** and the excitation light beam **81** into one exposure-excitation light beam in case of concurrence of these beams.

The shutter **73** of the exposure light beam **71** is used to let through the exposure light beam **71** in a controlled manner. Moreover, this shutter **73** is provided with a time lock, whereby it is interconnected by a time bond (in Fig. 1 it is indicated by a dashed line) with the shutter **83** of the excitation light beam **81,** thus enabling to achieve, for example, the required delay of the exposure light beam **71** in relation to the excitation light beam **81,** or, optionally, a different predetermined time sequence of these light beams **71, 81**, and in this manner to set appropriately the fatigue loading of the photochromic, fluorescent or phosphorescent dye/dyes, or of the mixture of at least two of them in the sample **3** into a suitable phase of its/their response to the excitation beam **81.**

The correction filter/filters **74** then serves/serve to filter off certain components of no interest or ballast components of the exposure light beam **71** and to approximate this beam, for example, to the character of natural daylight at particular times of the day, or to the character of another kind of light, according to required conditions of testing. If the character of the exposure light beam **71** meets the requirements of the fatigue testing, the correction filter/filters **74** can be omitted from the construction of the device.

The source **72** of the exposure light beam **71** is a polychromatic light source which simulates (optionally in cooperation with a correction filter/correction filters **74**) the required illumination of the sample **3** according to the particular requirements of fatigue testing, for example, natural daylight, etc., and which in this manner serves to carry out fatigue testing of the photochromic, fluorescent or phosphorescent dye/dyes, or of the mixture of at least two of them in the sample **3.**

The shutter **83** of the excitation light beam **81** is used to transmit the excitation light beam **81** in a controlled manner according to the needs and the setting of the fatigue testing, and in cooperation with the chopper **781** it is used to control the course of the phase of the color response of the photochromic, fluorescent or phosphorescent dye/dyes or of the mixture of at least two of them in the sample **3.**

The monochromator **84** is intended to change/adjust the wavelength of the excitation light beam **81** according to the character of the tested photochromic, fluorescent or phosphorescent dye or their mixture. Using different settings when performing the testing of one sample **3** allows to carry out an analysis of the spectral sensitivity of the particular photochromic, fluorescent or phosphorescent dye/dyes or the mixture of at least two of them.

The source **82** of the excitation light beam **81** is a source of the excitation light beam **82** which evokes the excitation of the photochromic, fluorescent or phosphorescent dye/dyes or of the mixture of at least two of them contained in the sample **3** and its possible color response. The parameters of this source **82** and its excitation light beam **81** depend on the spectral sensitivity of the dye/dyes being tested, whereby, for example, the light source used in the illustrated example of embodiment is a source illuminating materials in the spectrum of UV radiation.

To the outlet aperture **7** of the measuring light beam **41** from the optical integrator **1** is outside the optical integrator **1** assigned a path **90** of the measuring light beam **41** with a spectrometer **94.** This path includes an objective **91,** a chopper **92** and mirror optics **93.** In addition, the outlet aperture **7** of the measuring light beam **41** is provided with an unillustrated elimination aperture which is used for eliminating chromatic aberration.

The objective **93** serves to focus on the active spot of the sample **3,** where the color response of its dye/dyes takes place and/or to adjust the size of the area of the sample **3** which is sensed by the spectrometer **94.** If the active spot of the sample **3** is sufficiently large and is suitably placed in relation to the outlet aperture **7** of the measuring light beam **41**, it is not necessary to use the objective **93.** However, if the path of the measuring light beam **41** is composed at least partially of optical fibers, it is always necessary to use the objective **81.**

It is advantageous if the chopper **84** is interconnected by a time bond (in Fig. 1 indicated by a dashed line) with a chopper **781** in the common portion **78** of the path of the exposure and excitation light beams **71**, **81**. At the same time, it can be used for a number of purposes according to the requirements and the course of the test, such as for generating a prismatic signal, which by using Fourier Transform enables to eliminate noise, for synchronizing the signal captured by the spectrometer **94** with the exposure and/or excitation light beam **71**, **81**, etc., by which means it contributes not only to achieving more accurate results of the fatigue testing and increases possibilities of using them, but also enables to modulate the time of sensing by the spectrometer **94** according to the needs and the character of the tested photochromic, fluorescent or phosphorescent dye/dyes or of the mixture of at least two of them.

The mirror optics **93,** composed of a mirror or system of mirrors, serves to direct and concentrate the measuring light beam **41,** reflected from the sample **3,** into the inner space of the spectrometer **82** without decreasing its intensity or changing its characteristics.

The spectrometer **82,** which is a standard spectrometer **82** of any known type, is used for detecting and evaluating spectral data of the tested photochromic, fluorescent or phosphorescent dye/dyes or of the mixture of at least two of them contained in the sample **3** with time dependence.

In order to exclude the so-called drift of the luminous sources, the device for fatigue testing of photochromic, fluorescent or phosphorescent dye/dyes or of a mixture of at least two of them according to the invention is preferably designed as a two-beam device. This means that it includes an unillustrated source of a reference light beam and to it assigned reference spectrometer, which measuries the reflection of the reference light beam from the constant reference sample having a known value of absolute reflectance, which is a white standard or, for example, a part of the white inner surface of the optical integrator.

The device for fatigue testing of photochromic, fluorescent or phosphorescent dye/dyes or of a mixture of at least two of them in the sample **3** is in the illustrated example of embodiment enclosed in a thermostatic box **10,** which is equipped with known unillustrated means of regulating the temperature, enabling to set the temperature of the sample **3** and its surroundings. In this manner, the thermostatic box **10** allows to monitor the influence of the temperature on the fatigue testing of photochromic and/or fluorescent and/or phosphorescent dye/dyes, or, on the contrary, to exclude totally the influence of the temperature or its change on the fatigue testing. In other unillustrated examples of embodiment, the thermostatic box can be minimized just for the purpose of storing the sample **3** and, possibly, its closest surroundings, or it is not used at all. In the vicinity of the sample **3,** for example below it, as is shown in Fig. 1, a thermostatic head **100** can be mounted for checking and/or controlling the temperature of the sample **3** and its surroundings.

The function of the device for fatigue testing of photochromic, fluorescent or phosphorescent dye/dyes of or a mixture of at least two of them consists in that due to the shutter **73** of the exposure light beam **71,** the shutter **83** of the excitation light beam **81,** their optional connection by a time bond, and the chopper **781,** the dye being tested is exposed cyclically according to predetermined requirements to luminous exposure , the particular dye is subject to fatigue loading at least by one luminous exposure or a cycle of exposures to the exposure light beam **71,** which simulates, for example, natural daylight, or some other light, as is required. The exposure and excitation light beams **71, 81** can be introduced into the inner space of the optical integrator **1** gradually in any time sequence (it is advantageous if the excitation light beam **81** is introduced first), or they can be made into one exposure-excitation beam.

Simultaneously, the measuring light beam **41** is also introduced into the inner space of the optical integrator. It is then introduced onto the sample **3** by being reflected from the inner wall of the optical integrator **1** and being reflected from the sample **3** it is carried onto the mirror optics **93** into the sensing space of the spectrometer **94** through the objective **91** and the chopper **92.** At the same time, the spectrometer **94** detects and records the characteristics of the measuring light beam **41,** or their change and/or the course of their change. The color response of the tested photochromic, fluorescent or phosphorescent dye/dyes or of the mixture of at least two of them causes the change of the characteristics of the measuring light beam **41,** whereby other changes in its characteristics are also caused by possible fatigue of the tested dye/dyes, the change of color response being connected to it. The data set obtained by the spectrometer **94** then according to the requirements and setting of the device illustrates the course of the color response, or, for example, only some of its phases during the fatigue loading of the particular photochromic, fluorescent or phosphorescent dye/dyes or the mixture of at least two of them, and so from this change and/or course of the change it is possible to deduce the course of the color response and/or the change in the color response of the particular photochromic, fluorescent or phosphorescent dye/dyes, or of the mixture of at least two of them in the sample **3** as a result of its exposure by the excitation light beam **81,** and thus the fatigue of the photochromic, fluorescent or phosphorescent dye/dyes, or of the mixture of at least two of them.

Furthermore, the cycles of fatigue loading by the exposure light beam **71** and/or by cycles of excitation of the color response by the excitation light beam **81** can be regular or irregular according to the requirements and can be set substantially in any mutual relation, as will be needed.

Also, this method for fatigue testing of photochromic, fluorescent or phosphorescent dye/dyes or of a mixture of at least two of them can take place in a continuous manner during any predetermined period of time.

### List of references

- 1: optical integrator
- 2: measuring aperture
- 3: sample
- 4: inlet aperture of the measuring light beam
- 41: measuring light beam
- 42: source of the measuring light beam
- 51: filter of luminous radiation having an excitation wavelength
- 52: filter of IR radiation
- 6: inlet aperture of the exposure and excitation light beams
- 7: outlet aperture of the measuring light beam
- 70: path of the exposure light beam
- 71: exposure light beam
- 72: source of the exposure light beam
- 73: shutter of the exposure light beam
- 74: correction filter
- 78: common part of the path of the exposure and the path of the excitation light beam
- 780: beam splitter
- 781: chopper
- 782: mirror optics
- 80: path of the excitation light beam
- 81: excitation light beam
- 82: source of the excitation light beam
- 83: shutter of the excitation light beam
- 84: monochromator
- 90: path of the measuring light beam
- 91: objective
- 92: chopper
- 93: mirror optics
- 94: spectrometer
- 10: thermostatic box
- 100: thermostatic head

## Claims

1. A method for fatigue testing of photochromic, fluorescent or phosphorescent dye/dyes or of a mixture of at least two of them, wherein a sample (3) containing photochromic, fluorescent or phosphorescent dye/dyes or a mixture of at least two of them is exposed to a predetermined number of cycles of luminous exposure to an excitation light beam (81), which evokes a color response of photochromic, fluorescent or phosphorescent dye/dyes or a mixture of at least two of them in the sample (3), whereby before and/or during and/or after each predetermined exposure to the excitation light beam (81) the sample (3) containing photochromic, fluorescent or phosphorescent dye/dyes or a mixture of at least two of them is exposed at least once to irradiation by an exposure light beam (71) from a polychromatic light source (72), due to which the dye/dyes is/are subject to fatigue loading, simultaneously, a measuring light beam (41), is introduced to the sample (3) and is reflected from it, whereby the change and/or the course of the change in the characteristics of the measuring light beam (41) reflected from the sample is monitored by a spectrometer (94), and from this change and/or the course of the change, the course of the color response and/or the change in the color response of the particular photochromic, fluorescent or phosphorescent dye/dyes or the mixture of at least two of them of the sample (3) to the exposure to an excitation light beam (81) is deduced, and thus the fatigue of this photochromic, fluorescent or phosphorescent dye/dyes or the mixture of at least two of them is also deduced.

2. The method according to Claim 1, **characterized in that the** exposure light beam (71) simulates natural daylight.

3. A device for fatigue testing of photochromic, fluorescent or phosphorescent dye/dyes or of a mixture of at least two of them containing a spherical optical integrator (1), in which a measuring aperture (2), an inlet aperture (4) of the measuring light beam (41), an inlet aperture (6) of an exposure and excitation light beams (71, 72) and an outlet aperture of the measuring light beam (7) are formed, whereby in the vicinity of the measuring aperture (2), outside the optical integrator (1), is arranged space for storing and fixing a sample (3) with the tested photochromic, fluorescent or phosphorescent dye/dyes or the mixture of at least two of them, to the inlet aperture (4) of the measuring light beam (41) is assigned outside the optical integrator (1) a source (42) of the measuring light beam (41), whereby in the path of the measuring light beam (41) is located a filter (51) of luminous radiation of excitation wavelength and a filter (52) of IR radiation, **characterised in that** to the inlet aperture (6) of the exposure and excitation light beams (71, 81) is outside the optical integrator (1) assigned a path (70) of the exposure light beam (71) containing a polychromatic light source (72) of the exposure light beam (71), and the path (80) of the excitation light beam (81) containing a source (82) of the excitation light beam (81), whereby both these paths (70, 80) have a common part (78), which contains a beam splitter (780), arranged inversely, a chopper (781) and mirror optics (782) for introducing the exposure and excitation light beams (71, 81) into the inner space of the optical integrator (1), wherein the path (70) of the exposure light beam (71) contains a shutter (73) of the exposure light beam (71), while the path (80) of the excitation light beam (81) contains a shutter (83) of the excitation light beam (81) and a monochromator (84), and to the outlet aperture (7) of the measuring light beam (41) is outside the optical integrator (1) assigned a path (90) of the measuring light beam (41) with a spectrometer (94), which contains a chopper (92) and mirror optics (93) for introducing the measuring light beam (41) into the sensing space of the spectrometer (94).

4. The device according to Claim 3, **characterized in that** the path (70) of the exposure beam (71) includes at least one correction filter (74).

5. The device according to Claim 3 or 4, **characterized in that** the path (90) of the measuring light beam (41) includes an objective (91).

6. The device according to any of Claims 3 to 5, **characterized in that** the chopper (781) in the common portion (78) of the path of the exposure and excitation light beams (71, 81) and the chopper (92) in the path (90) of the measuring beam (41) are interconnected by a time bond.

7. The device according to any of Claims 3 to 6, **characterized in that** the shutter (73) of the exposure light beam (71) is provided with a time lock and is interconnected with the shutter (83) of the excitation light beam (81) by a time bond.

8. The device according to any of Claims 3 to 7, **characterized in that** all its components are mounted in a thermostatic box (10).

9. The device according to any of Claims 3 to 7, **characterized in that** the space for storing and fixing the sample (3) is located in the thermostatic box (10).

10. The device according to any of Claims 3 to 9, **characterized in that** in the vicinity of the space for storing and fixing the sample (3) is mounted a thermostatic head (100).

11. The device according to any of Claims 3 to 9, **characterized in that** at least part of the path (70) of the exposure light beam (71) and/or at least part of the path (80) of the excitation light beam (81) and/or at least part of the path (90) of the measuring light beam (41) is composed of an optical fiber/optical fibers.

12. The device according to any of Claims 2 to 11, **characterized in that** it contains a source of reference light beam, with which a reference spectrometer is aligned.

## Patentansprüche

1. Verfahren zur Ermüdungsprüfung von Photochrom-, Leucht- oder Leuchtphosphorfarbstoff/-farbstoffen, oder Mischung von mindestens zwei von ihnen, **bei dem** die Probe (3), die Photochrom-, Leucht- oder Leuchtphosphorfarbstoff/- farbstoffe oder Mischung von mindestens zwei von ihnen enthält, einer im Voraus festgelegten Anzahl von Belichtungszyklen durch einen Exzitationslichtstrahl (81) ausgesetzt wird, der eine Farbreaktion des/der Photochrom-, Leucht- oder Leuchtphosphorfarbstoffs/-farbstoffe oder der Mischung von mindestens zwei von ihnen der Proben (3) hervorruft, wobei vor und/oder während und/oder nach jeder im Voraus gewählten Belichtung durch einen Exzitationslichtstrahl (81) die Probe (3), die Photochrom-, Leucht- oder Leuchtphosphorfarbstoff/-farbstoffe oder Mischung von mindestens zwei von ihnen enthält, mindestens einmal der Belichtung durch einen Expositionslichtstrahl (71) aus einer polychromatischen Lichtquelle (72) ausgesetzt wird, die ihren Farbstoff/ihre Farbstoffe ermüdungsbezogen beansprucht, wobei an die Probe (3) zugleich ein Messlichtstrahl (41) zugeführt wird, der von ihr reflektiert wird, und mittels eines Spektrometers (94) Änderung und/oder Verlauf der Änderung der Charakteristik des Messlichtstrahls (41) verfolgt werden, der von der Probe (3) reflektiert wird, und aus dieser Änderung und/oder diesem Verlauf der Änderung auf Verlauf der Farbreaktion und/oder Änderung der Farbreaktion des/der jeweiligen Photochrom-, Leucht- oder Leuchtphosphorfarbstoffs/-farbstoffe oder Mischung von mindestens zwei von ihnen der Proben (3) auf die Belichtung durch den Exzitationslichtstrahl (81), und dadurch auf die Ermüdung des/der Photochrom-, Leucht- oder Leuchtphosphorfarbstoffs/-farbstoffe, oder der Mischung von mindestens zwei von ihnen geschlussfolgert wird.

2. Verfahren nach dem Anspruch 1, **dadurch gekennzeichnet, dass** der Expositionslichtstrahl (71) das natürliche Tageslicht simuliert.

3. Einrichtung zur Ermüdungsprüfung von Photochrom-, Leucht- oder Leuchtphosphorfarbstoff/-farbstoffen oder Mischung von mindestens zwei von ihnen, die einen optischen Integrator (1) aufweist, in dem Messöffnung (2), Eingangsöffnung (4) des Messlichtstrahls (41), Eingangsöffnung (6) des Expositions- und Exzitationslichtstrahls (71, 72) und Ausgangsöffnung des Messlichtstrahls (7) gebildet sind, wobei in der Nähe der Messöffnung (2) außerhalb des optischen Integrators (1) ein Raum zur Lagerung und Sicherstellung der Probe (3) mit dem/den zu prüfenden Photochrom-, Leucht- oder Leuchtphosphorfarbstoff/-farbstoffen oder Mischung von mindestens zwei von ihnen angeordnet ist, an die Eingangsöffnung (4) des Messlichtstrahls (41) ist außerhalb des optischen Integrators (1) eine Quelle (42) des Messlichtstrahls (41) angeschlossen, wobei in der Bahn des Messlichtstrahls (41) ein Filter (51) der Lichtstrahlung mit einer Exzitationswellenlänge und ein Filter (52) der IR-Strahlung angeordnet sind, **dadurch gekennzeichnet, dass** der Eingangsöffnung (6) des Expositions- und Exzitationslichtstrahls (71, 81) außerhalb des optischen Integrators (1) die Strahlenbahn (70) des Expositionslichtstrahls (71) mit der Quelle (72) des Expositionslichtstrahls (71), und die Strahlenbahn (80) des Exzitationslichtstrahls (81) mit der Quelle (82) des Exzitationslichtstrahls (81) zugeordnet sind, wobei beide dieser Strahlenbahnen (70, 80) einen gemeinsamen Teil (78) aufweisen, der einen Strahlteiler (780) in einer inversen Anordnung, Chopper (781) und Spiegeloptik (782) zur Zuführung des Expositions- und Exzitationslichtstrahls (71, 81) in den inneren Raum des optischen Integrators (1) aufweist, wobei die Strahlenbahn (70) des Expositionslichtstrahls (71) einen Verschluss (73) des Expositionslichtstrahls (71) aufweist, und die Strahlenbahn (80) des Exzitationslichtstrahls (81) einen Verschluss (83) des Exzitationslichtstrahls (81) und Monochromator (84) aufweist, und der Ausgangsöffnung (7) des Messlichtstrahls (41) außerhalb des optischen Integrators (1) die Strahlenbahn (90) des Messlichtstrahls (41) mit einem Spektrometer (94) zugeordnet ist, die Chopper (92) und Spiegeloptik (93) zur Zuführung des Messlichtstrahls (41) in den Aufnahmeraum des Spektrometers (94) aufweist.

4. Einrichtung nach dem Anspruch 3, **dadurch gekennzeichnet, dass** in der Strahlenbahn (70) des Expositionslichtstrahls (71) mindestens ein Korrektionsfilter (74) angeordnet ist.

5. Einrichtung nach dem Anspruch 3 oder 4, **dadurch gekennzeichnet, dass** in der Strahlenbahn (90) des Messlichtstrahls (41) ein Objektiv (91) angeordnet ist.

6. Einrichtung nach einem der Ansprüche 3 bis 5, **dadurch gekennzeichnet, dass** der Chopper (781) im gemeinsamen Teil (78) der Strahlenbahn des Expositions- und Exzitationslichtstrahls (71, 81) und Chopper (92) in der Strahlenbahn (90) des Messlichtstrahls (41) durch eine zeitliche Kopplung gekoppelt sind.

7. Einrichtung nach einem der Ansprüche 3 bis 6, **dadurch gekennzeichnet, dass** der Verschluss (73) des Expositionslichstrahls (71) mit einen zeitlichen Schloss versehen ist und durch eine zeitliche Verkopplung mit dem Verschluss (83) des Exzitationslichtstrahls (81) gekoppelt ist.

8. Einrichtung nach einem der Ansprüche 3 bis 7, **dadurch gekennzeichnet, dass** alle von ihren Teilen in einer thermostatischen Box (10) gelagert sind.

9. Einrichtung nach einem der Ansprüche 3 bis 7, **dadurch gekennzeichnet, dass** der Raum zur Lagerung und Fixation der Probe (3) in einer thermostatischen Box (10) gelagert ist.

10. Einrichtung nach einem der Ansprüche 3 bis 9, **dadurch gekennzeichnet, dass** in der Nähe des Raumes zur Lagerung und Sicherstellung der Probe (3) ein thermostatischer Kopf (100) gelagert ist.

11. Einrichtung nach einem der Ansprüche 3 bis 9, **dadurch gekennzeichnet, dass** mindestens ein Teil der Strahlenbahn (70) des Expositionslichtstrahls (71) und/oder mindestens ein Teil der Strahlenbahn (80) des Exzitationslichtstrahls (81) und/oder mindestens ein Teil der Strahlenbahn (90) des Messlichtstrahls (41) durch optische Faser/Fasern gebildet werden.

12. Einrichtung nach einem der Ansprüche 2 bis 11, **dadurch gekennzeichnet, dass** sie eine Quelle des Referenzlichtstrahls aufweist, dem ein Referenzspektrometer zugeordnet ist.

## Revendications

1. Procédé de tester la fatigue d'un colorant (des colorants) photochromique, fluorescent ou phosphorescent, ou d'un mélange d'au moins deux d'entre eux, dans lequel l'échantillon (3) contenant un colorant (des colorants) photochromique, fluorescent ou phosphorescent, ou un mélange d'au moins deux d'entre eux, il est soumis à un nombre prédéterminé de cycles d'excitation par un faisceau lumineux d'excitation (81) qui produit une réponse colorée du colorant (des colorants) photochromique, fluorescent ou phosphorescent ou un mélange d'au moins deux d'entre eux de l'échantillon (3), tandis qu' avant et / ou pendant et / ou après chaque exposition présélectionnée par faisceau lumineux d'excitation (81), un échantillon (3) contenant un colorant (des colorants) photochromique, fluorescent ou phosphorescent ou un mélange d'au moins deux d'entre eux est soumis à au moins une exposition par un faisceau lumineux d'exposition (71) à partir d'une source de lumière polychromatique (72) qui fatigue son colorant (ses colorants), tandis que à l'échantillon (3) est simultanément introduit un faisceau de lumière de mesure (41), qui est reflété par lui, et par le spectromètre (94) on surveille le changement et / ou le cours du changement de la caractéristique du faisceau lumineux (41) réflété de l'échantillon (3), et de ce changement et / ou du cours du changement on juge le cours de la réponse de couleur et / ou le changement de la réponse de couleur d'un colorant (des colorants) photochromique, fluorescent ou phosphorescent donné ou d'un mélange d'au moins deux d'entre eux de l'échantillon (3) à l'illumination par le faisceau de lumière d'excitation (81), et ainsi à la fatigue de ce colorant (de ces colorants) photochromique, fluorescent ou phosphorescent, ou d'un mélange d'au moins deux d'entre eux.

2. Procédé selon la revendication 1, **caractérisé en ce que** le faisceau de lumière d'exposition (71) simule la lumière du jour naturelle.

3. Dispositif pour les tests de fatigue d'un colorant (des colorants) photochromique, fluorescent ou phosphorescent ou d'un mélange d'au moins deux d'entre eux, comprenant un intégrateur optique sphérique (1) dans lequel il y a une ouverture de mesure (2), une ouverture d'entrée (4) du faisceau de lumière de mesure (41), une ouverture d'entrée (6) du faisceau de lumière d'exposition et d'excitation (71, 72) et une ouverture de sortie du faisceau de lumière de mesure (7), tandis que dans la proximité de l'ouverture d'entrée (2) en dehors de l'intégrateur optique (1) il y a l'espace pour stocker et sécuriser l'échantillon (3) avec un colorant testé (des colorants testés) photochimique, fluorescent ou phosphorescent, ou un mélange d'au moins deux d'entre eux, à l'ouverture d'entrée (4) du faisceau lumineux de mesure (41) en dehors de l'intégrateur optique (1) il y a la source (42) du faisceau lumineux de mesure (41), tandis que le filtre (51) du rayonnement lumineux avec la longueur d'excitation des ondes (51), et le filtre (52) du rayonnement IR sont inclus dans la trajectoire du faisceau lumineux de mesure (41) **caractérisé en ce qu'**avec l'ouverture d'entrée (6) du faisceau lumineux d'exposition et d'excitation (71, 81) à l'extérieur de l'intégrateur optique (1) le guidage (70) du faisceau lumineux d'exposition (71) avec la source (72) du faisceau lumineux d'exposition et le guidage (80) du faisceau lumineux d'excitation (81) avec la source (82) du faisceau lumineux d'excitation (81) sont associés, tandis que les deux guidage (70, 80) ont une partie commune qui comprend un diviseur du faisceau (780) en arrangement inverse, une gaine (781) et une optique de miroir (782) pour amener le faisceau lumineux d'exposition et d'excitation (71, 81) dans le circuit interne de l'intégrateur optique (1), tandis que le guidage (70) du faisceau lumineux d'exposition (71) comprend la fermeture (73) du faisceau lumineux d'exposition (71), et le guidage (80) du faisceau lumineux d'excitation (81) comprend une fermeture (83) du faisceau lumineux d'excitation (81) et un monochrome (84), et à l'extérieur de l'intégrateur optique (1) le guidage (90) du faisceau lumineux de mesure (41) est associé avec l'ouverture de sortie (7) du faisceau lumineux de mesure (41) avec un spectromètre (94) qui comprend une gaine (92) et une optique à miroir (93) pour amener le faisceau lumineux de mesure (41) dans la zone du balayage du spectromètre (94).

4. Dispositif selon la revendication 3, **caractérisé en ce qu'**au moins un filtre de correction (74) est inclus dans le guidage (70) du faisceau lumineux d'exposition (71).

5. Dispositif selon la revendication 3 ou 4, **caractérisé en ce que** dans le guidage (90) du faisceau lumineux de mesure (41) une lentille (91) est incluse.

6. Dispositif selon l'une quelconque des revendications 3 à 5, **caractérisé en ce que** la gaine (781) dans la partie commune (78) du faisceau lumineux d'exposition et d'excitation (71, 81) et la gaine (92) dans le guidage (90) du faisceau lumineux de mesure (41) sont liées par un lien temporel.

7. Dispositif selon l'une quelconque des revendications 3 à 6, **caractérisé en ce que** la fermeture (73) du faisceau lumineux d'exposition (71) est équipée par le verrouillage temporel et elle est couplée par le lien temporel avec la fermeture (83) du faisceau lumineux d'excitation (81).

8. Dispositif selon l'une quelconque des revendications 3 à 7, **caractérisé en ce que** tous ses composants sont stockés dans une boîte thermostatique (10).

9. Dispositif selon l'une quelconque des revendications 3 à 7, **caractérisé en ce que** l'espace pour le stockage et la fixation de l'échantillon (3) est stocké dans une boîte thermostatique (10).

10. Dispositif selon l'une quelconque des revendications 3 à 9, **caractérisé en ce qu'**une tête thermostatique (100) est disposée à proximité de l'espace pour le stockage et pour la fixation de l'échantillon (3).

11. Dispositif selon l'une quelconque des revendications 3 à 9, **caractérisé en ce qu'**au moins une partie du faisceau lumineux d'exposition (71) et / ou au moins une partie du faisceau lumineux d'excitation (81) et / ou au moins une partie du guidage (90) du faisceau lumineux de mesure (41) est formée par le fibre/ les fibres optique/s.

12. Dispositif selon l'une quelconque des revendications 2 à 11, **caractérisé en ce qu'**i**l** comporte la source d'un faisceau lumineux de référence auquel est associé un spectromètre de référence.
